(19) **Europäisches Patentamt / European Patent Office / Office européen des brevets**

(11) **EP 4 744 585 A1**

(12) # EUROPEAN PATENT APPLICATION

(43) Date of publication:
20.05.2026 Bulletin 2026/21

(21) Application number: 25215479.4

(22) Date of filing: 13.11.2025

(51) International Patent Classification (IPC):
**A61B 5/06** (2006.01)  **A61B 5/00** (2006.01)
**A61N 1/365** (2006.01)  **A61N 1/372** (2006.01)

(52) Cooperative Patent Classification (CPC):
**A61N 1/372; A61B 5/068; A61B 5/7264;**
**A61N 1/36514; A61N 1/36521; A61N 1/37205;**
**A61N 1/37235; A61N 1/37241; A61N 1/3756**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: 13.11.2024  US 202463720171 P
12.11.2025  US 202519386701

(71) Applicant: **Pacesetter, Inc.**
**Sylmar, CA 91342 (US)**

(72) Inventors:
- **Johnson, Eric**
  **Sylmar, CA 91342 (US)**
- **Guthrie, John Clark**
  **Sylmar, CA 91342 (US)**
- **Huff, Christine C**
  **Sylmar, CA 91342 (US)**
- **Bruhns, Kenneth P**
  **Sylmar, CA 91342 (US)**
- **Saucerman, James Drew**
  **Sylmar, CA 91342 (US)**

(74) Representative: **Barker Brettell Sweden AB**
**Kungsbroplan 3**
**112 27 Stockholm (SE)**

(54) **SYSTEMS FOR USE DURING LEADLESS PACEMAKER IMPLANT PROCEDURE**

(57)     A system (100) and computer readable medium for use during an implant procedure during which a delivery system (100) is being used to implant a leadless pacemaker (LP) (102a, 102b) within a patient may, while the delivery system (100) is being used to position the LP (102a, 102b) at a potential implant site within the patient: receive, from the LP (102a, 102b), data comprising one or more pacing impedance measurements and at least one measurement obtained by the LP (102a, 102b) other than pacing impedance; provide the data to a model (950) that is produced prior to the implant procedure based on data collected from other LPs implanted in other patients; and use the model (950) to output an indication of whether the LP (102a, 102b) should be chronically implanted at the potential implant site.

FIG. 2

EP 4 744 585 A1

**Description**

FIELD OF TECHNOLOGY

Embodiments described herein generally relate leadless pacemakers (LPs) capable of delivering pacing, as well as external systems that communicate with LPs. Embodiments described herein also relate to systems for use during an LP implant procedure.

BACKGROUND

Over the past few years leadless pacemakers (LPs) have started being implanted in patients in place of conventional pacemakers. LPs may eliminate the need for implanting leads. This is beneficial because leads may fail and/or migrate more often than desired. There is also an aesthetic benefit of not having a pulse generator placed in a subcutaneous pocket.

The selection of an implantation site of an LP is typically based on a clinician's judgement and experience as to whether a site is a viable site for chronically implanting the LP.

After a period of time (e.g., three or more months) following an LP implant procedure it may become apparent that the implantation site is not optimal. In some cases this results in poor performance (e.g., excess battery usage) or the need for a follow up surgical procedure to reposition the pacemaker. Subjecting a patient to such a follow up surgical procedure is undesirable.

SUMMARY

Embodiments include systems and non-transitory computer readable medium for use during an implant procedure used to implant an LP within a patient, the LP including a fixation mechanism and a distal electrode located at a distal end of the LP and also including a further electrode. The implant procedure includes one or more mapping portions and one or more testing portions, wherein during each mapping portion of the implant procedure the distal electrode is in contact with the potential implant site and the fixation mechanism is not affixed to the potential implant site. The system may include a receiver configured to wirelessly receive, from the LP, data comprising one or more pacing impedance measurements and at least one measurement obtained by the LP other than pacing impedance. The system may include, and one or more processors communicatively coupled to the receiver. The one or more processors is/are configured to provide at least a portion of the data received by the receiver to a model that is produced prior to the implant procedure based on data collected from other LPs implanted in other patients. The one or more processors is/are also configured to use the model to output an indication of whether the LP should be chronically implanted at a potential implant site. A method may include or use other or different equipment.

Embodiments may improve pacemaker technology by allowing an LP to work more effectively, by being implanted at a site where the LP is less likely to have to be re-sited (aka repositioned) during a follow up surgical procedure, and/or by allowing the LP to use less battery power and thereby increase its longevity. Embodiments may also improve the LP's treatment of heart conditions.

BRIEF DESCRIPTION OF THE DRAWINGS

Non-limiting examples of embodiments of the disclosure are described below with reference to figures attached hereto. Dimensions of features shown in the figures are chosen for convenience and clarity of presentation and are not necessarily shown to scale. The subject matter regarded as the invention is particularly pointed out and distinctly claimed in the concluding portion of the specification. The invention, however, both as to organization and method of operation, together with objects, features, and advantages thereof, can be understood by reference to the following detailed description when read with the accompanied drawings. Embodiments are illustrated without limitation in the figures, in which like reference numerals may indicate corresponding, analogous, or similar elements, and in which:

FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 illustrates a high level flow diagram showing a method for use during an implant procedure .
FIG. 5 shows example p-values for various measurements obtained during a mapping portion and a testing (e.g., tether) phase or portion of an implant procedure, and which measurements can be provided to one or more of the models described herein.
FIGS. 6 and 7 illustrate, respectively, example receiver operating characteristic (ROC) curves produced based on current of injury (COI) and pacing impedance measures obtained during the mapping portion of an implant procedure, and during the testing (e.g., tether) portion of an implant procedure.

FIG. 8 illustrates an example of a portion of an EGM sensed by an LP during an implant procedure with some of the features thereof labeled, wherein measurements of such features may be determined and provided to one of the models described herein.

FIG. 9 shows a block diagram of one embodiment of an external device for use in communicating with and/or programming an LP during an implant procedure, and which can be used to implement certain embodiments of the present invention.

FIG. 10 illustrates a high level flow diagram showing a method of creating and utilizing a model during an implant procedure.

DETAILED DESCRIPTION

In the following detailed description, numerous specific details are set forth in order to provide a thorough understanding of various embodiments of the invention. However, it will be understood by those skilled in the art that certain embodiments of the present invention can be practiced without these specific details. In other instances, well-known methods, procedures, components, modules, units and/or circuits have not been described in detail so as not to obscure the embodiments of invention described herein.

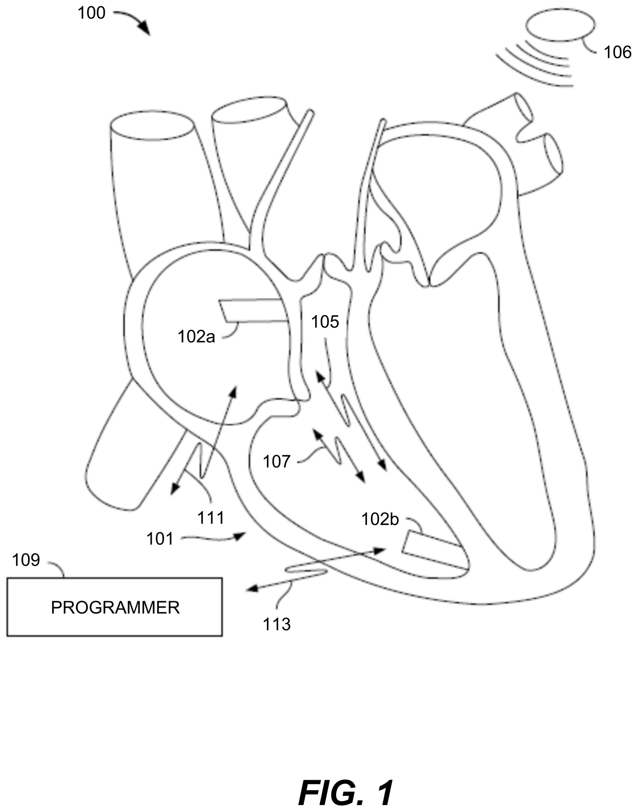

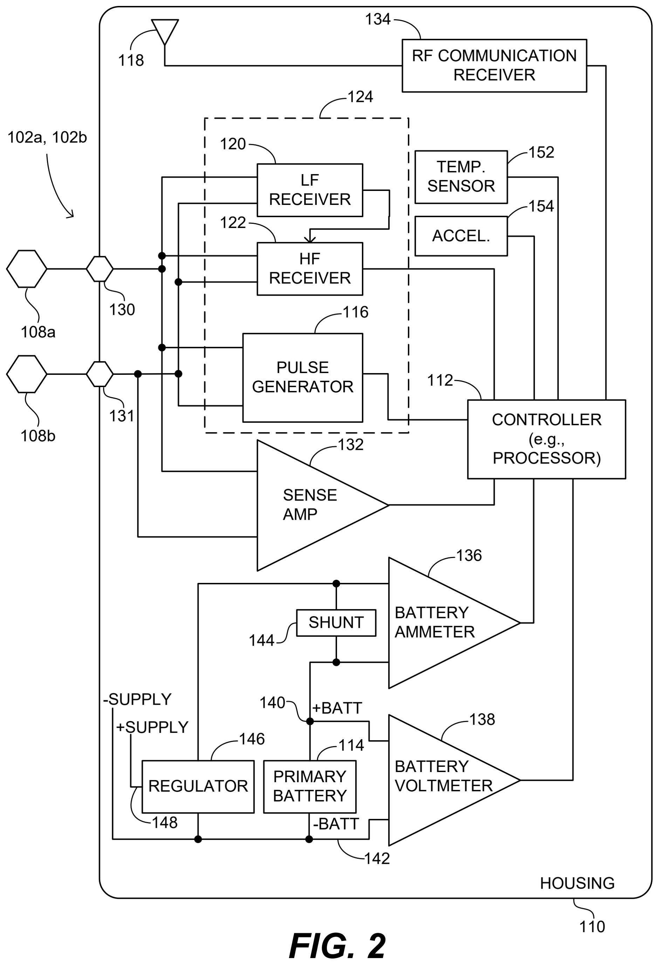

An LP typically includes two or more leadless electrodes (e.g., electrodes 108a, 108b, in FIGS. 1-3) configured for delivering cardiac pacing pulses and sensing evoked and/or natural (aka intrinsic) cardiac electrical signals. The electrodes can also be used for performing unidirectional or bidirectional conductive communication, if the LP is configured to perform conductive communication. One of the electrodes (e.g., electrode 108a), which may be referred to as the distal or tip electrode, can be located on a distal end of the LP. Another electrode (e.g., electrode 108b), which may be referred to as the can or ring electrode, can be located on a housing (e.g., housing 110, 202 in FIGS. 2-3) of the LP and may be considered the proximal electrode. While LPs, and a specific fixation mechanism, are discussed herein as an example, other types of pacemakers and fixation mechanisms may be used with embodiments of the present invention. For example, it is also possible that an LP includes more than two electrodes.

For an LP to induce a response in excitable myocardial tissue, a cardiac pacing pulse should have a sufficient combination of pulse amplitude and pulse duration to initiate a cascade of self-propagating depolarizing wavefronts in the heart to "capture" local myocardial tissue. This minimal stimulus intensity, used to cause capture, is known as a pacing capture threshold (PCT), or more succinctly as the capture threshold. An LP typically sets its pacing stimulation level to exceed the capture threshold by a safety margin. For example, a safety margin can be applied by multiplying a pacing voltage (corresponding to the capture threshold) by a multiplier, or a safety margin can be applied by adding a voltage to a pacing voltage (corresponding to the capture threshold).

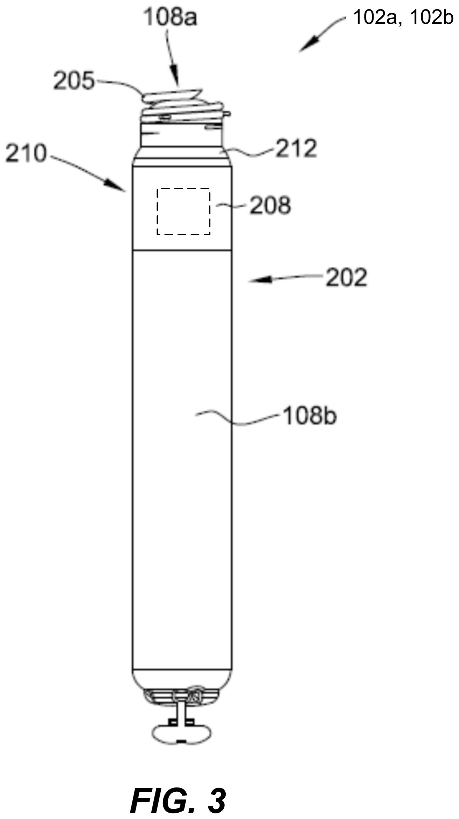

An LP can include a fixation mechanism (e.g., fixation mechanism 205 in FIG. 3) at its distal end (proximate to the distal electrode 108a) and an attachment feature at its proximal end. The fixation mechanism located at the distal end of the LP can include a fixation helix, one or more of hooks, barbs, and/or one or more other fixation features configured to affix the LP to heart tissue. The attachment feature located at the proximal end of the LP can be used during an implant procedure and can be configured to be selectively attached to a portion of a delivery system so that the delivery system can be used to implant the LP at an implant site. The implant system may include a delivery catheter (not shown) that is used to deliver the LP to the implant site. The delivery system may also include one or more tethers (not shown) that may be introduced through a lumen of the delivery catheter and used during a tether mode (aka test mode) to test a pacing capture threshold, among other reasons. Such a tether may be, without limitation, a snare, a flexible shaft, a cable, and/or the like.

During a mapping phase or portion (aka mapping mode) of an implant procedure the delivery catheter is used to position a distal portion of the LP against cardiac wall tissue at a potential implant site (without affixing the LP to the potential implant site) and the LP is used to obtain one or more pacing impedance measurements and sense an electrogram (EGM) or other measurements, such as PCT. Additionally, during the mapping phase or mode, the LP transmits data inclusive of the pacing impedance measurement(s) and the EGM to an external device, such an external programmer or another device external to the patient's body, that is configured to wirelessly communicate with the LP, for example using a receiver to wirelessly receive data from an LP. The pacing impedance measurement(s) and the EGM can be displayed on a display of (or communicatively coupled to) the external device, such as external programmer, and used by a clinician or physician to determine whether they believe, anecdotally, heuristically, and/or based on past experience, whether the potential implant site appears to be a viable site for chronically implanting the LP. If the clinician or physician believes the potential implant site appears to be a viable site for chronically implanting the LP, they can then manipulate (e.g., rotate) at least a portion of the delivery catheter to affix the fixation mechanism (e.g., a fixation helix) to the implant site and more specifically to cardiac tissue of a cardiac wall. While the LP is affixed to the cardiac tissue the distal electrode of the LP should be in contact with the cardiac tissue. Thereafter the delivery catheter can be maneuvered such that the LP is separated from the delivery catheter, but the LP is still coupled to one or more tethers of the delivery system.

During this portion of the implant procedure, which can be referred to as the testing portion (aka testing mode) or the tether portion (aka tether mode) of the implant procedure, the LP is freely supported by one or more tethers without additional support from the delivery catheter of the delivery system. During the testing portion of the implant procedure the LP can obtain one or more pacing impedance measurements and sense an EGM. During the testing portion of the implant procedure the LP can also determine a pacing capture threshold for the LP at the present implant site. Additionally, during the testing portion of the implant procedure the LP can transmit data including the pacing impedance measurement(s), the EGM, and the pacing capture threshold (for the LP at the present implant site) to the external device, such as external programmer. The pacing impedance measurements, EGM and pacing capture threshold can be displayed on the display of (or communicatively coupled to) the external device, such as external programmer, and used by the clinician or physician to determine whether they believe, anecdotally, heuristically, and/or based on past experience, that the LP should remain at the present implant site or should be unaffixed from the cardiac tissue so that a better implant site can be found. If the clinician or physician determines that the LP should not remain at the present implant site, they can use the delivery system to unaffix the LP from the cardiac tissue and to reposition the distal portion of the LP against cardiac wall tissue at a new potential implant site. The clinician or physician can repeat the above described mapping mode and thereafter the above described tether mode until the clinician or physician determines that the LP should remain at an implant site. If the clinician or physician determines that the LP should remain at the present implant site then the tether(s) are detached from the LP, and more generally, the delivery system is completely detached from the LP. The delivery system can then be removed from the patient.

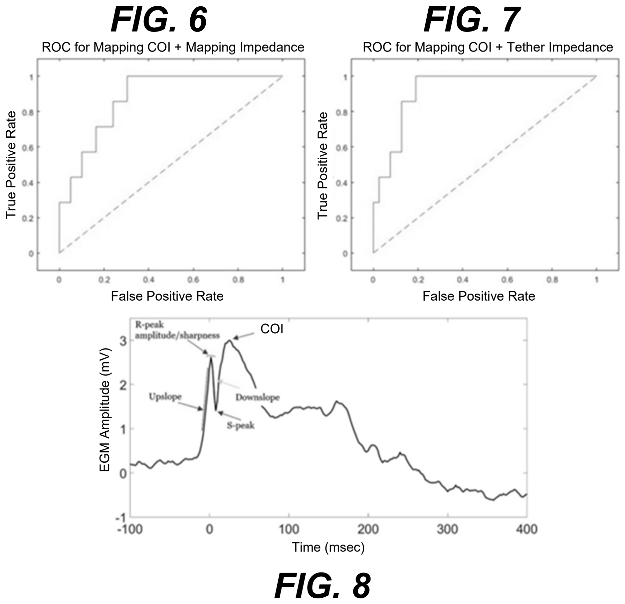

Among the measurements, data or features that may be obtained by the LP and/or the external device, such as external programmer, during mapping and/or testing phases is the current of injury (COI), which may be the ST-segment elevation shown in the EGM when the distal electrode of the LP contacts with endocardium. The COI may be a peak voltage elevation in an EGM (e.g. as shown in the example in Fig. 8), for example following the S-wave, and may be measured by an automatic process reviewing an EGM, a technician or doctor reviewing an EGM, or a combination thereof (e.g. a user indicating on an EGM display a peak amplitude value to be used as COI).

When implanting an LP using a fixation mechanism (e.g., a fixation helix, one or more of hooks, barbs, etc.) that is configured to affix the LP to heart tissue (aka myocardial tissue, or cardia tissue), the heart tissue undergoes a certain amount of localized injury while the fixation mechanism is implanted. The localized injury is generally referred to as the current of injury (COI). The COI represents the flow of current to or from an injured tissue region of the heart due to regional alteration of the transmembrane potential at the point of fixation. The COI is recognized at the site of tissue injury as an increase in the duration of the EGM and/or in the elevation of the ST segment following a QRS complex. An example measure of the COI may be the peak amplitude of an EGM immediately following a QRS complex. However, additional and/or alternative measures of the COI may be used, as is known in the art.

After a period of time (e.g., three or more months) following an LP implant procedure the pacing capture threshold for the LP may increase for a myriad of reasons, not all of which are known. A significant increase in the pacing capture threshold is undesirable because it may prevent the LP from being able to effectively pace the cardiac chamber in or on which the LP is implanted. An increase in the pacing capture threshold is also undesirable because it may require the LP to increase its pacing stimulation energy (e.g., its pacing pulse amplitude and/or pulse width), which will result in a battery (e.g., battery 114 in FIG. 2) of the LP being depleted more rapidly than would be the case if the pacing capture threshold did not increase, thereby reducing the longevity (e.g., lifespan) of the LP.

During a patient's follow up visit to a clinician or physician, and/or based on remote monitoring of the pacing capture threshold of the LP, a clinician or physician may determine that the pacing capture threshold has increased to the point that it is necessary to have a follow up surgical procedure to reposition the LP so that the LP is affixed to cardiac tissue at another implant site that allows for a lower pacing capture threshold. Subjecting a patient to such a follow up surgical procedure is undesirable because it subjects the patient to potential risks (e.g., of an infection, of an adverse reaction to anesthesia, and/or the like) as well as because the follow up surgical procedure is inconvenient and expensive.

Embodiments of the present invention can be used during an initial implant procedure to assist a clinician or physician with selecting an implant site at which to affix an LP within a patient that reduces a probability that the LP will need to be repositioned at a later time. Accordingly, such embodiments can reduce a probability that the patient will be subjected to an undesirable follow up surgical procedure.

In accordance some embodiments, empirical data (e.g., ground truth data representing a pacemaker or heart measurement data which can be used to evaluate the success of the placement) is collected from each of a plurality of patients having one or more implanted LPs (e.g., at the time of implant as well as one or more later points in time, e.g., at three-month follow up visits by the patients and/or collected using a remote monitoring system). The empirical data (e.g. data collected during mapping, tethering and implantation associated with post-implantation data such as collected after several months of implantation) may be used to produce one or more models that are configured to be used during an implant procedure to determine for a newly seen patient, based on data received from an LP during the implant procedure for the newly seen patient, whether a potential implant site is more likely than not a viable site for chronically implanting the LP, and if not, that the LP should be repositioned during the implant procedure such that another implant site is used.

In one embodiment, the post-implantation metric (e.g., three-month metric) measured is PCT, which can be used alone or in conjunction with another mechanism (e.g., a threshold) to determine if the implant site, if in the past, resulted in a viable implantation, or if output by a model, if the candidate or potential implant site is predicted to result in a viable implantation. One or more metrics or ground truth data other than and/or in addition to PCT may be used. In accordance with certain embodiments, such a model is configured to be used by an external device (e.g., an external programmer) during an implant procedure to predict a pacing capture threshold of an LP at a time in the future (e.g., three months in the future, but not limited thereto) based on data collected by the LP during the implant procedure and transmitted to the external device. Such data can be transmitted by the LP to the external device (e.g., the external programmer) using conductive communication, or alternatively using another type of wireless communication technology, such as radio frequency (RF) communication or inductive communication. The data received by the external device (e.g., the external programmer) is used by the model to make one or more predictions relating to whether a potential implant site is a viable site for chronically implanting the LP, and if not, to for example indicate that the LP should be repositioned during the implant procedure such that another implant site is used. The external device (e.g., the external programmer) can also be referred to more generally herein as an external system.

An embodiment may use features such as EGM and impedance as inputs to predict a single output such as a mathematical probability of a measure of success, or PCT at three-months, which may be used in a linear (e.g., direct) regression model, such as in the example in Table 2 below, or a logistic regression (e.g., by converting the PCT into a binary value) model, such as in the example in Table 1 below. While in an example embodiment PCT and placement are assessed, other outcomes may be assessed by embodiments of the present invention. For example, LP dislodgement or cardiac wall perforation (which are by nature binary outcomes) may be assessed by embodiments, via a binary logistic regression model. Another related outcome to be predicted by certain embodiments includes a system revision, which involves another procedure in which the LP is retrieved and replaced or just deactivated and replaced. Other adverse events may alternatively or additionally be modeled. The various example formulas described herein, e.g., in Tables 1, 2 and 3, may be adapted, for example with different coefficients, to predict such different outcomes. While a three month timeframe as the measure of likely good pacemaker placement is discussed herein, in other embodiments, different timeframes (e.g. one-month, six-months, etc.) for PCT may be used, or an embodiment may predict change in PCT over time and use this as an indication of likely good or bad placement, using ML, AI or other models as discussed herein.

An exemplary system in or with which embodiments of the present technology can be used will first be described with reference to FIGS. 1-3. More specifically, FIGS. 1-3 will be used to describe an example dual chamber LP system, which optionally also includes another implantable medical device (IMD), for instance a non-vascular implantable cardioverter defibrillator (NV-ICD), such as a subcutaneous-ICD (S-ICD), and an external device (e.g., an external programmer). Technology other than shown in FIGS. 1-3, including different implantable systems, and different external systems, may be used with embodiments of the present invention.

FIG. 1 illustrates a system 100, sometimes referred to as cardiac pacing system herein, including components configured to be implanted in a heart 101. The system 100 includes LPs 102a and 102b located in different chambers of the heart. LP 102a is located in a right atrium, and thus can also be referred to herein as an atrial LP (aLP). LP 102b is located in a right ventricle, and thus can also be referred to herein as a ventricular LP (vLP). The aLP 102a and vLP 102b can be referred to collectively herein as the LPs 102, or individually as an LP 102. The LPs 102a and 102b can communicate with one another to inform one another of various local physiologic activities, such as local intrinsic events (e.g. the heart's natural and healthy electrical activity), local paced events and the like. The LPs 102a and 102b may be constructed in a similar manner, but operate differently (e.g. based on programming or control of controller 112 in FIG. 2) based upon which chamber the LP 102a or 102b is located. While two LPs are illustrated in FIG. 1, embodiments of the present invention can be used to assist with implanting a single LP 102 in a patient, two LPs 102 in a patient, or more than two (e.g., three or four) LPs in a patient. Controller or processor 112, e.g. one or more microchips, CPUs, etc., including or in combination with memory devices and other chips, may control the operation of the LP 102.

In certain embodiments, LPs 102a and 102b communicate with one another, and/or with an IMD, such as an ICD 106, by conductive communication through the same electrodes that are used for sensing and/or delivery of pacing therapy. LPs 102a and 102b may also be able to use conductive communication and those same electrodes to communicate with an external device, e.g., an external programmer 109, having electrodes placed on the skin of a patient within which the LPs 102a and 102b are implanted. The LPs 102a and 102b can each alternatively, or additionally, include an antenna that would enable them to communicate with one another, the ICD 106 and/or an external device, using radio (RF) communication. Alternatively, or additionally, it is possible that LPs 102a, 102b utilize another type of communication, such as inductive communication, in which case the LPs 102a, 102b can each include a respective inductive communication coil. While only two LPs are shown in FIG. 1, it is possible that more than two LPs can be implanted in a patient. For example, to provide for bi-ventricular pacing and/or cardiac resynchronization therapy (CRT), in addition to having LPs implanted in the right atrial (RA) chamber and the right ventricular (RV) chamber, a further LP can be implanted in the left ventricular (LV) chamber.

Programmer 109 may be a device or computer system external to the patient allowing a user to interface with a pacemaker, including LP 102, internal to the patient. Such a device may be in communication with a pacemaker being implanted, may perform analysis such as executing a model to determine the suitability of an implantation site, display the results of a model, display data from a pacemaker or a patient's heart such as an EGM, allow an operator to enter commands, and perform other functions. Each LP 102 may use two or more electrodes located within, on, or within a few centimeters of the housing of the LP, for pacing and sensing at the cardiac chamber. Where the LPs 102 communication using conductive communication, the electrodes of the LPs 102 can also be used for bidirectional conductive communication with one another, as well as with the programmer 109, and the ICD 106.

Referring to FIG. 2, a block diagram shows an embodiment for portions of the components and electronics within LPs 102a, 102b configured to provide conductive communication through the sensing/pacing electrodes 108a, 108b. One or more of LPs 102a and 102b include at least two leadless electrodes configured for delivering cardiac pacing pulses, sensing evoked and/or natural cardiac electrical signals, and unidirectional or bi-directional conductive communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation.

In FIG. 2, each of the LPs 102a, 102b is shown as including first and second receivers 120 and 122 that collectively define separate first and second conductive communication channels 105 and 107 (FIG. 1), (among other things) between LPs 102a and 102b. The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 100 KHz) and the receiver 122 is configured to monitor for one or more signals within a relatively high frequency range (e.g., above 100 KHz). In certain embodiments, the receiver 120 (and more specifically, at least a portion thereof) is always enabled and monitoring for a wakeup notice, which can simply be a wakeup pulse, within a specific low frequency range (e.g., between 1 KHz and 100 KHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 100 KHz), but consumes significantly less power than the high power receiver 122. This way the low power receiver 120 is capable of always monitoring for a wakeup notice without significantly depleting the battery (e.g., 114) of the LP. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery of the LP (which the high power receiver 122 may do if it were always enabled). Since the receivers 120, 122 are used to receive conductive communication messages, the receivers 120, 122 can also be referred to as conductive communication receivers. In certain embodiments, an LP includes only a single conductive communication receiver. Although first and second receivers 120 and 122 are depicted, in other embodiments, each LP 102a, 102b may only include the first receiver 120, or more generally may include only a single receiver that is configured to receive conductive communication signals. It is also possible that an LP 102 may include additional receivers other than first and second receivers 120 and 122. The pulse generator 116 can function as a transmitter that transmits implant-to-implant (i2i) communication signals using the electrodes 108.

In accordance with certain embodiments, when one of the LPs 102a and 102b senses an intrinsic event or delivers a paced event, the corresponding LP 102a, 102b transmits an implant event message to the other LP 102a, 102b. For example, when an aLP 102a senses/paces an atrial event, the aLP 102a transmits an implant event message including an event marker indicative of a nature of the event (e.g., intrinsic/sensed atrial event, paced atrial event). When a vLP 102b senses/paces a ventricular event, the vLP 102b transmits an implant event message including an event marker indicative of a nature of the event (e.g., intrinsic/sensed ventricular event, paced ventricular event). In certain embodiments, each LP 102a, 102b transmits an implant event message to the other LP 102a, 102b preceding the actual pace pulse so that the remote LP can blank its sense inputs in anticipation of that remote pace pulse (to prevent inappropriate crosstalk sensing). The above described implant event messages are examples of i2i messages.

Still referring to FIG. 2, the LP 102 is also shown as including an antenna 118 that is coupled to a radio frequency (RF) communication transceiver 134, which is configured to transmit and receive RF communication messages using an RF communication protocol, such as a Bluetooth® protocol, WiFi protocol, Bluetooth® low energy (BLE) protocol, Medical Device Radiocommunications Service (MedRadio) protocol, and/or the like. In certain embodiments, the antenna 118 can be integrated into a fixation mechanism (e.g., fixation mechanism 205 in FIG. 3) of the LP 102, in which case the antenna can be referred to as a fixation antenna. The RF communication transceiver 134 may consume more battery power than the conductive communication transceiver 124. More generally, it may be more power efficient for an LP 102 (or other type of IMD 106) to use conductive communication than to use RF communication to communicate with another LP 102 (or other type of IMD 106). Accordingly, in accordance with certain embodiments of the present invention, the LP 102 (or other type of IMD 106) is configured to primarily transmit and receive messages using conductive communication, and RF communication is used as a backup or auxiliary type of communication that can be used when conductive communication is deactivated (aka turned off) or is unsuccessful or otherwise deficient, as will be described in additional detail below.

In some embodiments the LPs 102a, 102b are only capable of utilizing conductive communication for communicating with one another and/or other devices (such as the programmer 109 and/or ICD 106), in which case the antenna 118 and the RF communication transceiver 134 may be eliminated. In some embodiments LPs 102a, 102b are only configured to communicate using RF communication, and to not utilize conductive communication, in which case certain circuitry may be eliminated, such as the receivers 120, 122. Alternatively, or additionally, it is possible that the LPs 102a, 102b utilize another type of communication, such as inductive communication, in which case the LPs 102a, 102b can each include a respective inductive communication coil.

In accordance with certain embodiments herein, the external device 109 may communicate with LP 102a, 102b utilizing one or more of the above described communication schemes, i.e., using conductive communication, RF communication, and/or inductive communication.

In some embodiments, each individual LP 102 can comprise a hermetic housing 110 configured for placement on or attachment to the inside or outside of a cardiac chamber and at least two leadless electrodes 108a, 108b proximal to the housing 110 and configured for bidirectional communication with at least one other device (e.g., the programmer 109 or the ICD 106) within or outside the body.

FIG. 2 depicts a single LP 102 (e.g., the LP 102a or 102b) and shows the LP's functional elements substantially enclosed in a hermetic housing 110. The LP 102 has at least two electrodes 108a, 108b located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108a, 108b, receivers 120, 122 for receiving information from at least one other device via the electrodes 108a, 108b, and a pulse generator 116 for generating pacing pulses for delivery via the electrodes 108a, 108b and also for transmitting information to at least one other device via the electrodes 108a, 108b. The housing 110 can optionally contain circuits for monitoring device health, for example an optional battery current monitor 136 and an optional battery voltage monitor 138, and can contain circuits for controlling operations in a predetermined manner. While one pulse generator 116 is shown in FIG. 2, it is possible that that LP includes multiple pulse generators, one of which is used for producing pacing pulses, and another one of which is used for producing conductive communication pulses. The pulse generator 116 that produces pacing pulses can include a charge pump to provide for a wide range of pacing pulse amplitudes, as will be described in additional details below.

The electrodes 108a, 108b can be configured to communicate bidirectionally among the multiple LPs 102a, 102b and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108a, 108b can be configured to communicate bidirectionally among the one or more LPs 102 and/or the ICD 106 and transmit data including designated codes for events detected or created by an individual pacemaker. Individual pacemakers can be configured to issue a unique code corresponding to an event type and a location of the sending pacemaker.

Referring again to FIGS. 1 and 2, the cardiac pacing system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the one or more LPs 102a, 102b configured for leadless intercommunication by information conduction through body tissue and/or wireless transmission between transmitters and receivers in accordance with the embodiments discussed herein.

In a further embodiment, a cardiac pacing system 100 comprises at least one LP 102a, 102b configured for implantation in electrical contact with a cardiac chamber and configured to perform cardiac pacing functions in combination with the co-implanted ICD 106. Each LP 102 may include at least two leadless electrodes 108a, 108b configured for delivering cardiac pacing pulses, sensing evoked and/or natural cardiac electrical signals, and transmitting information to the co-implanted ICD 106.

As shown in the illustrative embodiments, an LP 102a, 102b can comprise two or more leadless electrodes 108a, 108b configured for delivering cardiac pacing pulses, sensing evoked and/or natural cardiac electrical signals, and bidirectionally communicating with the co-implanted ICD 106.

Each LP 102a, 102b can be configured for operation in a respective particular location and to have a respective particular functionality at manufacture and/or by programming by an external programmer 109. Bidirectional communication among the multiple LPs 102a, 102b can be arranged to communicate notification of a sensed heartbeat or delivered pacing pulse event and encoding type and location of the event to another implanted pacemaker or pacemakers. The LP 102a, 102b receiving the communication decode the information and respond depending on location of the receiving pacemaker and predetermined system functionality.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in which the LP 102a, 102b is implanted (and thus with which the LP 102a, 102b is interacting; e.g., pacing and/or sensing). Some non-limiting examples include an evoked response algorithm, use of atrial fibrillation (AF) suppression in a local chamber, blanking and refractory periods, etc. Accordingly, each LP 102a, 102b should know an identity of the chamber in (or on) which the LP 102a, 102b is implanted, and processes may be implemented to automatically identify a local chamber associated with each LP 102a, 102b.

Also shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the LP 102. The optional shunt 144 enables the optional battery current monitor 136 to provide the controller 112 with an indication of battery current drain and indirectly of device health. The illustrative power supply can be a primary battery 114. The battery 114 produces a battery voltage ($V_{battery}$) that can be measured, e.g., using the optional battery voltmeter 138. A charge pump of the pulse generator 116 can be used to produce pacing pulse of various different amplitudes based on the battery voltage, as described in additional detail below.

Still referring to FIG. 2, the LP 102 is shown as including an optional temperature sensor 152. The optional temperature sensor 152 can be any one of various different types of well-known temperature sensors, or can be a future developed temperature sensor. For one example, the optional temperature sensor 152 can be a thermistor, a thermocouple, a resistance thermometer, or a silicon bandgap temperature sensor, but is not limited thereto. Regardless of how the optional temperature sensor 152 is implemented, it is preferable that the temperature sensed by the optional temperature sensor 152 is provided to the controller 112 as a digital signal indicative of the blood temperature of the patient within which the LP 102 is implanted. The optional temperature sensor 152 can be hermetically sealed within the housing 110, but that need not be the case. The optional temperature sensor 152 can be used in various manners. For example, the optional temperature sensor 152 can be used to detect an activity level of the patient to adjust a pacing rate, i.e., for use in rate responsive pacing.

Referring to FIG. 2, the LP 102 is also shown as including an optional accelerometer 154 which can be hermetically contained within the housing 110. The optional accelerometer 154 can be any one of various different types of well-known accelerometers, or can be a future developed accelerometer. For one example, the optional accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the optional accelerometer 154 can be used to detect an activity level and/or posture of the patient to adjust a pacing rate, e.g., for use in rate responsive pacing. It would also be possible to use outputs of both the optional accelerometer 154 and the optional temperature sensor 152 to monitor the activity level of a patient.

In various embodiments, the LP 102 can manage power consumption to draw limited power from the battery 114, thereby reducing device volume. Each circuit in the LP 102 can be designed to avoid large peak currents. For example, cardiac pacing can be achieved by discharging a tank capacitor (not shown) across the electrodes 108a, 108b. Recharging of the tank capacitor is typically controlled by a charge pump circuit. In a particular embodiment, the charge pump circuit is throttled to recharge the tank capacitor at constant power from the battery 114. The charge pump circuit, which can be referred to more generally as the charge pump, is a type of DC-DC converter that leverages switched-capacitor techniques to either increase or decrease a voltage level that is output by the battery 114 of the LP 102. More generally, the charge pump can be used to adjust the voltage level ($V_{out}$) that is output by the battery 114 by one or more multiples, e.g., such as 0.5, 1.0, 1.5, 2.0, or 3.0. That is why charge pumps are sometimes referred to as multipliers. Such a charge pump can be part of the pulse generator 116, as noted above.

FIG. 3 shows an example form factor of the LPs 102a, 102b. Each LP 102a, 102b can include a hermetic housing 202 (e.g., housing 110 in FIG. 2) with electrodes 108a, 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the LP 102a, 102b to tissue, such as heart tissue. As noted above, an antenna (e.g., 118) can be at least partially implanted by or as part of the fixation mechanism. The electrodes 108a, 108b are examples of the electrodes 108a, 108b shown in and discussed above with reference to FIG. 2.

The housing 202 can also include an electronics compartment 210 within the housing 202 that contains the electronic components necessary for operation of the LP 102a, 102b, including, e.g., a pulse generator, transceiver, a battery, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator 208 disposed on the conductive material to separate the electrodes 108a, 108b. The insulator 208 can be an insulative coating on a portion of the housing 202 between the electrodes 108a, 108b, and can comprise materials such as silicone, polyurethane, parylene, or another biocompatible electrical insulator commonly used for implantable medical devices. In the embodiment of FIG. 3, a single insulator 208 is disposed along the portion of the housing 202 between the electrodes 108a, 108b. In some embodiments, the housing 202 itself can comprise an insulator instead of a conductor, such as an alumina ceramic or other similar materials, and the electrodes 108a, 108b can be disposed upon the housing 202.

As shown in FIG. 3, the LP 102a, 102b can further include a header assembly 212 to isolate the electrodes 108a, 108b. The header assembly 212 can be made from for example polyether ether ketone (PEEK), tecothane or another biocompatible plastic, and can contain a ceramic to metal feedthrough, a glass to metal feedthrough, or other appropriate feedthrough insulator as known in the art.

The electrodes 108a, 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A fixation mechanism 205, such as a helical fixation mechanism or device, can enable insertion of the device endocardially or epicardially through a guiding catheter. A torqueable catheter can be used to rotate the housing 202 and force the fixation device into heart tissue, thus affixing the fixation mechanism 205 (and also the electrode 108a in FIG. 3) into contact with stimulable tissue. Electrode 108b can serve as an indifferent electrode for sensing and pacing. The fixation mechanism 205 may be coated partially or in full for electrical insulation, and a steroid-eluting matrix may be included on or near the device to minimize fibrotic reaction, as is known in conventional pacing electrode-leads.

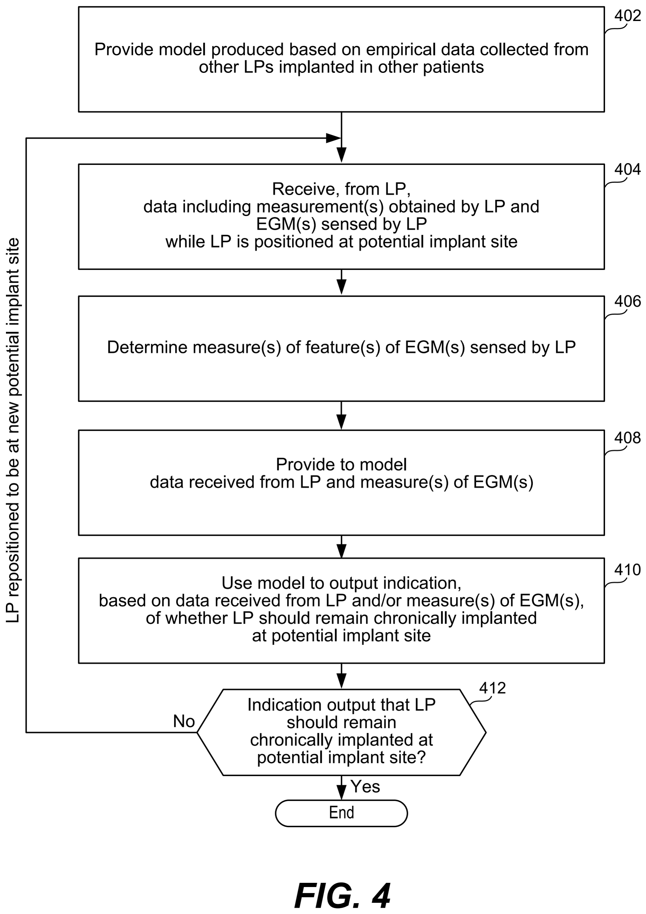

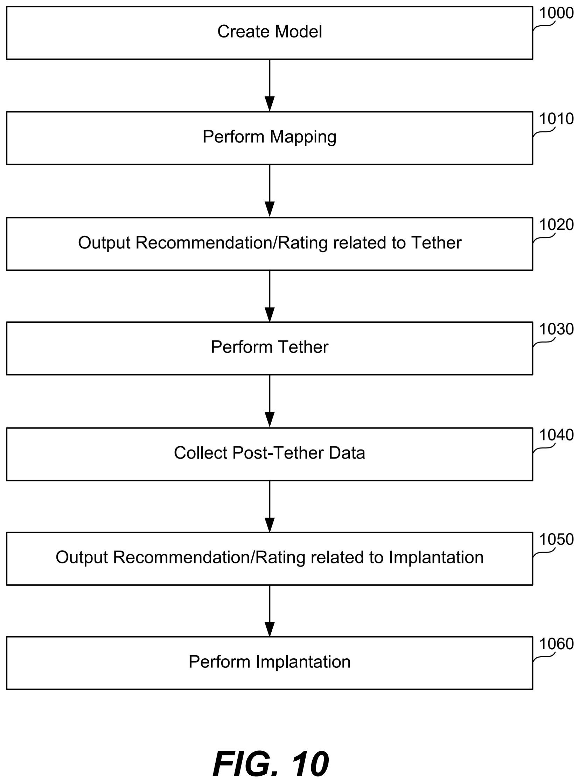

The high level flow diagram of FIG. 4 will now be used to describe example methods, which are for use during an implant procedure during which a delivery system (e.g., including a delivery catheter and one or more tethers) is being used to implant an LP within a patient. The example methods of FIGS. 4 and 10 may be used with devices and systems as depicted herein, but may be also used with other systems. As with other flowcharts herein, while an exemplary method is depicted for illustrative purposes in the flowchart of FIG. 4, it will be appreciated by those skilled in the art that features and operations from this procedure may be selectively combined with features and operations from alternative embodiments of the invention without departing from the remit of the disclosure. Further, while certain features and operations are expressly included in the flowcharts shown herein, it will be appreciated by those skilled in the art that not all depicted features and operations are mandatory elements, and that different embodiments may omit certain features or operations without departing from the remit of the disclosure. Accordingly, embodiments including combinations of the features and operations recited in the flowcharts shown herein are expressly within the remit of the disclosure and do not constitute an intermediate generalization of the same.

Referring to FIG. 4, in step 402 a model may be produced, such as trained, or provided based on empirical data collected from other LPs implanted in other patients. Step 402 can be performed prior to an implant procedure being performed for a patient for which the remaining steps in FIG. 4 are to be performed. The model can be for example a linear regression model, such as a univariate linear regression model or a multivariate linear regression model, but is not limited thereto. Alternatively, the model can be a logistic regression model. In still other embodiments, the model can be a machine learning (ML) model or artificial intelligence (AI) model that is trained based on the empirical data collected from the other LPs implanted in other patients. For example, a neural network (NN) trained on data as discussed herein may accept input signals associated with a potential implant site, such as those used in Table 1 or Table 2, and output an indication of suitability for the site. In certain embodiments, the model is a binary classifier model.

The remaining steps in FIG. 4, including steps 404, 406, 408, 410, and 412 (or a subset thereof) are performed during an implant procedure during which a delivery system (e.g., including a delivery catheter and one or more tethers) is being used to implant an LP within a patient. Such an implant procedure, as described herein, can include one or more mapping portions (aka mapping modes) and one or more testing portions (aka testing modes or tether modes). During each mapping portion of the implant procedure the distal electrode (e.g., electrode 108a) of the LP (e.g., LP 102) is placed in contact with a potential implant site, using the delivery system, and the fixation mechanism (e.g., fixation mechanism 205) is not affixed to the potential implant site. During each testing portion of the implant procedure the distal electrode (e.g., electrode 108a) of the LP (e.g., LP 102) is in contact with the potential implant site and the fixation mechanism (e.g., fixation mechanism 205) of the LP is affixed (and more specifically, has already been affixed) to the potential implant site using the delivery system. In accordance with certain embodiments, steps 404, 406, 408, 410, and 412 (or a subset thereof) are performed by an external system or device (e.g., an external programmer) that is configured to wirelessly communicate with the LP being implanted.

During the mapping portion of the implant procedure, the LP can (autonomously or under the controller of the external system) deliver pacing pulses while located at the potential implant site and measure the corresponding pacing impedance and determine a corresponding pacing capture threshold. For example, the LP can initially deliver pacing pulses having a high amplitude, e.g., 5 Volts (V), that is expected to cause capture (of the cardiac chamber in which the LP is position) and the LP may gradually reduce the amplitude of pacing pulses to determine the amplitude at which capture is lost, which provides a measure of the pacing capture threshold. When gradually decreasing the pacing amplitude, the capture threshold may be the lowest pacing amplitude at which capture was successful. Alternatively, the LP can initially deliver pacing pulses having a low amplitude, e.g., 0.1 V, not expected to cause capture (of the cardiac chamber in which the LP is position) and the LP may gradually increase the amplitude of pacing pulses to determine the amplitude at which capture is caused, which provides a measure of the pacing capture threshold. When gradually increasing the pacing amplitude, the capture threshold is similarly the lowest pacing amplitude at which capture was successful. The use of other techniques for determining a measure of a pacing capture threshold for the LP at a potential implant site are also possible and within the scope of the embodiments described herein. Additionally, during the mapping portion of the implant procedure the LP can sense an EGM.

Similarly, during the testing portion of the implant procedure, during which the LP (e.g., LP 102) is in contact with the potential implant site and the fixation mechanism (e.g., fixation mechanism 205) has already been affixed to the potential implant site using the delivery system, the LP can (autonomously or under the controller of the external system) deliver further pacing pulses while located at the potential implant site and measure the corresponding pacing impedance and determine a corresponding pacing capture threshold. Additionally, during the testing portion of the implant procedure the LP can sense another EGM. The measurements and sensed EGM obtained by the LP during the testing portion of the implant procedure will likely differ from the measurements and sensed EGM obtained by the LP during the mapping portion of the implant procedure, because during the testing mode the LP is affixed to the potential implant site, which was not the case during the mapping portion of the implant procedure.

Referring again to FIG. 4, step 404 includes the external system receiving, from the LP, data including one or more pacing impedance measurements obtained by the LP, one or more pacing capture threshold measurements obtained by the LP, and an electrogram (EGM) sensed by the LP. Some of the data that is received from the LP at step 404 may have been obtained by the LP during the mapping portion of the implant procedure, while other data that is received from the LP at step 404 may have been obtained by the LP during the testing portion of the implant procedure. Operations 404-410 may include, during an implant procedure during which a delivery system is being used to implant a LP within a patient, and while the delivery system is being used to position the LP at a potential implant site within the patient, receiving, from the LP, data including for example pacing impedance measurements and one or more measurements (e.g. obtained by the LP) other than pacing impedance.

Still referring to FIG. 4, step 406 includes determining (automatically, by the external system; by a user; or by a combination thereof) one or more measures of one or more features of the EGM(s) sensed by the LP. This can include determining measures of one or more current of injury (COI) features, one or more R-wave features, and/or one or more S-wave features. Measures of COI features can include, e.g., COI amplitude, COI area under the curve (AUC), and/or the like. Measures of R-wave features can include, e.g., R-wave amplitude, R-wave upslope, R-wave peak sharpness, R-wave downslope, R-wave AUC, and/or the like. Measures of S-wave features can include, e.g., S-wave amplitude, S-wave upslope, S-wave peak sharpness, S-wave downslope, S-wave AUC, and/or the like. Measure(s) of feature(s) of the EGM(s) sensed by the LP can be determined for the EGM sensed by the LP during the mapping portion of an implant procedure and/or for the EGM sensed by the LP during the testing portion of an implant procedure. The measure(s) of feature(s) of the EGM(s) sensed by the LP can, for example, be determined by the LP that sensed the EGM(s) and/or can determined by an external device (e.g., an external programmer 109) that receives EGM data from the LP.

Step 408 includes providing to the model at least some of the data received from the LP. For example, the collected data may be provided to a model that is produced prior to the implant procedure based on data collected from other patients; non-limiting examples of such models are shown in Tables 1, 2 and 3. This can include providing to the model a pacing impedance measurement obtained by the LP and a pacing capture threshold measurement obtained by the LP for the potential implant location during the mapping portion of the implant procedure. Additionally, or alternatively, this can include providing to the model a pacing impedance measurement obtained by the LP and a pacing capture threshold measurement obtained by the LP for the potential implant location during the testing portion of the implant procedure. Step 408 can also involve providing one or more measures of one or more features of the EGM sensed by the LP during the mapping portion of the implant procedure, and/or providing one or more measures of one or more features of the EGM sensed by the LP during the testing portion of the implant procedure.

Still referring to FIG. 4, step 410 includes using the model to output an indication, based on the data received from the LP, of whether the LP should be chronically implanted at the potential implant site. In certain embodiments, the model is a binary classifier type of model, and the indication output by the model (of whether the LP should be chronically implanted at the potential implant site) is a binary indication (e.g., a Yes or No indication). For example, the model can predict, based on the measurements and/or other data provided to the model at step 408, what the pacing capture threshold will be for the LP in the future (e.g., 3 months in the future, but not limited thereto) if the LP was chronically implanted at the potential implant site, and/or the model can determine whether the predicted pacing capture threshold in the future will be below a predetermined threshold. The predetermined threshold can be specified by default or can be programmed by a clinician or physician.

Alternatively, the indication output by the model (of whether the LP should be chronically implanted at the potential implant site) can be a probabilistic indication. In still other embodiments, the indication output by the model (of whether the LP should be chronically implanted at the potential implant site) can be a predicted PCT for the LP a period of time in the future (e.g., 3 months in the future) if the LP were chronically implanted at the potential implant site. A clinician or physician can then determine whether the predicted pacing capture threshold for the LP the period of time in the future is too high or is acceptable. A high pacing capture threshold at three months, or another time post-implant, may require repositioning of a LP; a high pacing capture threshold at a post-implant time may not require repositioning but may reduce LP battery life.

The indication output by the model, which specifies whether the LP should be chronically implanted at the potential implant site, can be a visual indication output via a display or touch screen, and/or can be an audio indication output via a speaker, but is not limited thereto.

Still referring to FIG. 4, at step 412 there is a determination of whether the model outputs an indication that the LP should be chronically implanted at the potential implant site. If the answer to the determination at step 412 is Yes, then the method ends, as shown in FIG. 4, and the delivery system can be removed from the patient and the LP can chronically remain affixed at the implant site. If instead the answer to the determination at step 412 is No, then a clinician or physician be use the delivery system to reposition the LP to be positioned at another (aka a new) potential implant site, and then the flow can return to step 404 and the above summarized steps can be repeated while the LP is positioned at the new potential implant site. After steps 404, 406, 408 and 410 (or a subset thereof) are repeated, at another instance of step 412 there is a determination of whether the model outputs an indication that the LP should be chronically implanted at the new potential implant site. This process can be repeated until the answer to the determination at an instance of step 412 is Yes, and the delivery system can be removed from the patient and the LP can chronically remain affixed at the most recently tested implant site. Embodiments may create a model for use in predicting the suitability of potential LP implantation sites. The model created may be for example a linear regression model, a univariate or multivariate linear regression model, a logistic regression model, an ML model, a binary classifier model, or other suitable models. The model may be created using or trained based on empirical data collected from "other" patients, e.g., patients other than a newly seen patient to whom the model is applied.

A model may be produced prior to the implant procedure based on (e.g. trained based on, or otherwise based on) data collected from patients other than the patient which is the subject of the implant procedure. In one embodiment, a model was created from a retrospective study of a leadless pacemaker clinical trial, including patients with data received from LPs, such as complete sets of impedance measurements and EGMs collected during the mapping portions and the testing portions of implant procedures, and pacing capture thresholds obtained at 3-month follow-ups. A computerized model was developed to quantify features of the EGM, including amplitudes of the R-wave, S-wave, and COI, the slope of the upstroke and downstroke, and the sharpness of the R-wave slope or peak (e.g. calculated as average slope of sampled points within $\pm 1$ of the peak). Linear regression was performed to identify significant predictors, among the data received from patients, of the chronic pacing capture threshold. Those significant predictors were used to create a model, such that the model uses those significant predictors. Binary logistic regression models were constructed by converting the 3-month pacing capture threshold measurements into a binary outcome using a cutoff of 1.5 V and analyzed using receiver operating characteristic (ROC) curves.

Data for eighty eight (88) patients were included in the retrospective study as input to a model created for one embodiment. The pacing capture thresholds at 3-months were $0.73 \pm 0.84$ V. Eight (8) patients had pacing capture thresholds greater than 1.5 V at 3 months. In univariate linear regression, among the data received from LPs, impedance during mapping and testing, the sharpness of the R-wave during mapping, and the R-wave amplitude during testing were found to be significant predictors of 3-month pacing capture threshold (p=0.04, <0.01, 0.05, 0.03, respectively). Two non-limiting embodiments including logistic regression models were identified and created: 1) using only mapping variables (COI and impedance), 2) including both mapping COI and tether impedance. The mapping logistic regression model included COI (p=0.01) and impedance (p=0.1) during mapping and produced an area under the curve (AUC) of 0.88 with sensitivity and specificity of 100% and 70%, respectively. A logistic regression model including COI (mapping, p=0.04) and impedance (tether, p=0.03) produced an AUC of 0.92 with sensitivity and specificity of 100% and 81%, respectively. Test of the $X^2$ statistic vs. constant model had p<0.01 in both models. Other methods of creating or training a model may be used. For example, all data received from LPs from a set of patients may be provided to a model such as a NN, or another model, and that model may determine which data received is significant for prediction of 3-month PCT, or a measure of success of implantation. The model may provide output as to which among the data is significant, and during an implant procedure only that data identified as significant may be used; alternatively, the model used during implant may receive all data received during training.

A computerized prediction model is created in one embodiment, for example, using intraoperative EGM and impedance to predict a pacing capture threshold for an LP 3-months following implant of the LP, which is useful in enhancing procedural efficacy and efficiency.

One embodiment uses a model using mapping COI (Map_COI) (e.g., a COI corresponding to an EGM obtained during a mapping phase) and testing impedance (Teth_Imp) (e.g., an impedance measurement obtained during a testing phase, aka tether phase), and the logistic regression equation in Table 1 below. Such a model may, for example, produce an AUC of 0.92 with sensitivity and specificity of 100% and 81%, respectively (as described in the example results above). An embodiment may receive LP data for a patient, input such data to a model including the formulas of Table 1 (the model produced prior to an implant for the patient and created based on data collected from other patients), and use the model to output an indication of whether the LP should be chronically implanted at the potential implant site associated with the received LP data:

$$Y' = 4.60 - 0.01004 \times Teth\_Imp - 0.804 \times Map\_COI$$

$$P(1) = \exp(Y')/(1+\exp(Y'))$$

Coefficients

Table 1

| Term | Coef SE | Coef | Z-Value | P-Value | VIF |
|---|---|---|---|---|---|
| Constant | 4.6 | 2.29 | 2.01 | 0.045 | |
| Teth_Imp | -0.01004 | 0.00474 | -2.12 | 0.034 | 1.00 |
| Map_COI | -0.804 | 0.401 | -2.01 | 0.045 | 1.00 |

In Table 1 above, P(1) is a logistical regression that outputs the mathematical probability of a given outcome (such as an undesirable outcome (e.g. PCT > 1.5V), or some measure of success), e.g. the probability (e.g., a probabilistic indication), from 0-1, of a candidate implant site being a successful implant site of the LP; Teth_Imp is a pacing impedance measurement during tether or testing (typically for a specific candidate site; typically one measurement, although averages or other aggregations of multiple measurements may be used); and Map_COI may be an amplitude of the COI during mapping (typically for a specific candidate site; typically one measurement, although averages or other aggregations of multiple measurements may be used). In the Coefficients listed, the first column describes the coefficients in the regression, and the remaining columns represent the statistical significance of the coefficients and their standard error (SE). While in the model of Table 1 mapping and tether data are used (as mapping and tether data may provide sufficient patient description, even if the LP is not in its final position), in another embodiment, post release data may be used. P(1) may represent a logistic regression to optimize data sets which are good implant sites and bad implant sites after three months of implantation, in a binary manner. The various inputs to the formulas shown in Tables 1, 2 and 3, and used in other embodiments, may be identified automatically by, e.g., an external device such as programmer 109. Other or different coefficients may be used.

An example optimal cutoff value from the above regression equation is 0.0797. In one embodiment patients with results above this cutoff are highly likely to develop a higher than desirable (e.g., >1.5 V) PCT by 3-months following implantation of an LP. This cutoff was calculated for one embodiment from the combination of a pacing impedance measurement (obtained by the LP during a testing phase) and a COI measurement (obtained by the LP during a mapping phase) and did not have a specific cutoff for either input independently. The cutoff value can also be tailored to prioritize sensitivity or specificity of the model. Tiers or ranges of values may also be utilized to maximize/optimize predictions.

Different embodiments may present different outputs to a user, who may use that output to proceed with a mapping, tethering, or and implant procedure. The formula in Table 1 may produce an output between 0 and 1, which may be compared to a threshold; e.g., if the value is below a threshold such as 0.0797, a positive (e.g., "implant") indication may be displayed, and if not, a negative (e.g., "do not implant") indication may be displayed. In other embodiments, the numeric output of the formula in Table 1 may be displayed, which may be used by the user to decide whether to continue mapping, to transition from mapping mode to tether mode, and/or to implant or not.

Additional inputs including measured, calculated or derived EGM or other cardiac features such as maximum amplitude of one or more features of the EGM can be used in other embodiments to create a model to further optimize the predictive capability, with a tradeoff of model and measurement simplicity verses incremental precision increases. The precision may not always increase with additional terms, however.

A model in some embodiments could further be applied to alternate patient outcomes, such as LP dislodgements, cardiac wall perforation by an LP, and/or other adverse events.

In one experiment, a set of 4 input variables (Mapping: R-wave sharpness and upslope, Testing: R-wave downslope; and impedance) was selected for a linear regression model with the example model and equation shown in Table 2. An embodiment may receive LP data for a patient, input such data to a model including the formulas of Table 2 (the model produced prior to an implant for the patient and created based on data collected from other patients), and use the model to output a predicted 3-month PCT and/or an indication of whether the LP should be chronically implanted at the potential implant site associated with the received LP data:

Table 2

Regression Equation
3-mo PCT = 1.725 - 0.001309×Teth_Imp - 0.001536×Map_R_med + 0.001826×Map_R_up + 0.000619×Teth_RS_slew

In the example regression formula of Table 2, Teth_Imp may be a pacing impedance measurement during tether or testing (typically one specific measurement, but in other embodiments averages or other aggregations of multiple measurements may be used), Map_R_med may be the average slope of the R-wave peak amplitude sharpness (e.g., as shown in Fig. 8; this may be the average or median of the slope of the upslope of this peak or close to the peak and the slope of the downslope of this peak or immediately following the peak), Map_R_up may be the slope of the upslope of the R-wave peak amplitude sharpness, and Teth_RS_slew may be the slope of the downslope of the R-wave (e.g. immediately following an R-wave peak), e.g., from the R-wave peak to the S-wave peak as in the example of FIG. 8. (The EGM shown in FIG. 8 is an example of an EGM which may be taken during tether, mapping, or any other period.) Typically, all inputs to the model of Table 2 are captured during the same heartbeat or small time period. The R-wave in an EGM may be a deflection after a Q-wave and is part of the QRS complex, and the R-wave may represent early ventricular depolarization. The QRS complex, generally, is the combination of the Q, R and S waves, corresponding to the depolarization of the right and left ventricles of the heart and contraction of the large ventricular muscles. The Q, R, and S-waves occur in rapid succession, and reflect a single event and thus are usually considered together. A Q-wave may be any downward deflection immediately following the P-wave. An R-wave may follow as an upward deflection, and the S-wave may be any downward deflection after the R-wave. The R-wave, and its upslope and downslope, and the slope of these features, may be identified by an automatic process reviewing an EGM (e.g. performing using one or more processors of an LP or external device), a user, technician or doctor reviewing an EGM and for example marking points of the ECM on a display for an external device to use in making a calculation or inputting values, or a combination thereof. For example, the upslope and down-slope of the R wave may be identified, for example, by a technician marking features on a screen, and/or an automatic process calculating the slope of identified features. The model of Table 2 may directly calculate the continuous variable output of chronic (3-month) PCT; this PCT may be directly output to a user. A model may input the 3 month PCT as shown in Table 2, compare it to one or more thresholds, and output an indication of the likelihood of success for an implantation site, or a recommendation as to whether a LP should be chronically implanted at a particular implantation site. Various values may be used as a threshold compared to a PCT, such as 1 V, 1.5 V, 2.5 V, etc., and may be determined based on, for example, battery chemistry and circuit design. For example, a step-up in capacitor filling may be used above a certain pacing amplitude: this may have a larger effect on battery life relative to lower steps.

Different embodiments may present different outputs to a user, who may use that output to proceed with a mapping, tethering, or and implant procedure. The formula in Table 2 produces predicted PCT at 3-months following implantation which may be compared to a cutoff or threshold; if the value is below a threshold, a positive (e.g. "implant") indication may be displayed, and if not, a negative (e.g. "do not implant") indication may be displayed. In other embodiments, the output of the formula in Table 2 may be displayed, which may be used by the user to decide if to continue mapping, to tether, to implant or not.

Various combinations of input variables may be selected from the possible EGM features (as well as impedance) to optimize the model's prediction. Intraprocedural pacing capture threshold may be used as another input variable in these models (similarly during mapping and testing portions).

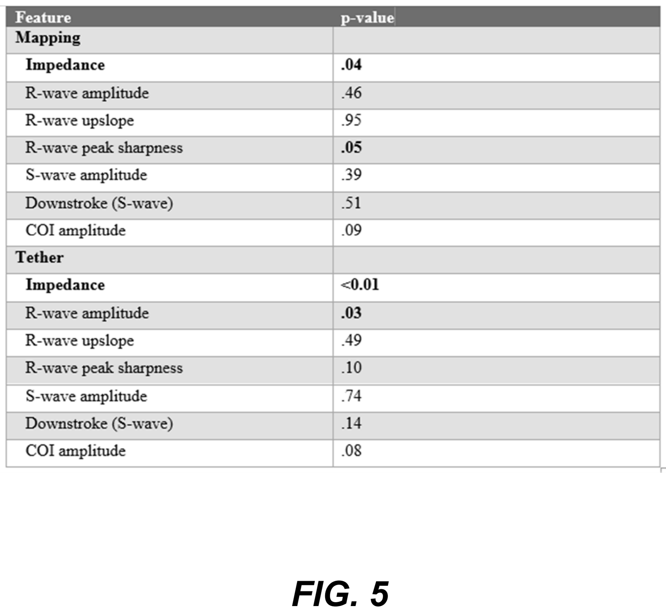

The table in FIG. 5 shows example p-values for various measurements obtained during a mapping portion and a testing (aka tether) portion of an implant procedure, and which measurements can be provided to one or more of the models described herein. Specifically, the values included in the table correspond to a model configured to provide univariate linear regression outcomes. Each variable is compared to the output result independently and significance of the regression shown as the p-value. In one example, a cutoff of 0.05 is used to determine significance such that a variable having a p-value at or below 0.05 p-value would be considered a significant predictor (e.g., is considered statistically significant).

FIGS. 6 and 7 illustrate, respectively, example receiver operating characteristic (ROC) curves produced based on COI and pacing impedance measures obtained during the mapping portion of an implant procedure, and during the testing (aka tether) portion of an implant procedure.

FIG. 8 illustrates an example of a portion of an EGM sensed by an LP during an implant procedure with some of the features thereof labeled, wherein measurements of such features may be determined and provided to one of the models described herein.

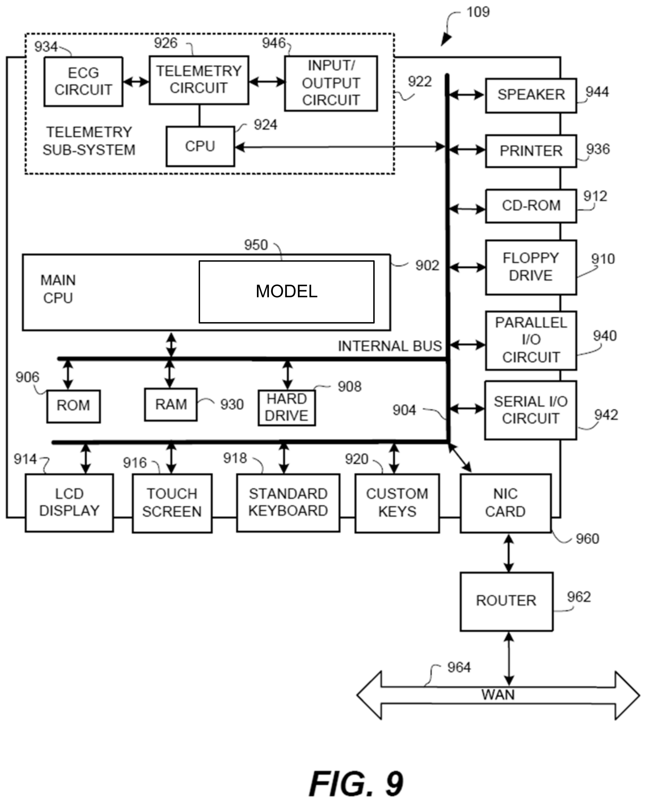

FIG. 9 illustrates example components of an example external device, system or programmer 109 for use in communicating with and/or programming an LP 102, and which can be used to perform one of the methods disclosed herein with reference to the flow diagrams of FIGS. 4 and 10. Further, the external device 109 may be capable of causing an LP 102 to perform functions necessary to complete certain methods of the present invention. The external device 109 can also be referred to as the external system 109.

Considering the components of the external device 109 by reference to FIG. 9, operations of the external device 109 can be controlled by a CPU 902, which may be a generally programmable microprocessor or microcontroller or may be a dedicated processing device such as an Application Specific Integrated Circuit (ASIC) or the like. Software instructions to be performed by the CPU 902 can be accessed via an internal bus 904 from a Read Only Memory (ROM) 906 and Random Access Memory (RAM) 930. Additional software may be accessed from a hard drive 908, floppy drive 910, and CD ROM drive 912, or other suitable permanent mass storage device. Depending upon the specific implementation, a Basic Input Output System (BIOS) is retrieved from the ROM 906 by CPU 902 at power up. Based upon instructions provided in the BIOS, the CPU 902 "boots up" the overall system in accordance with well-established computer processing techniques.

A non-transitory computer readable medium including or having stored thereon instructions executable by at least one processor (e.g. CPU or processor 902) may be for example one or more of ROM 906, RAM 930, hard drive 908, floppy drive 910, CD ROM drive 912, or other suitable storage devices. Embodiments may include such a non-transitory computer readable medium including or having stored thereon instructions executable by at least one processor to carry out methods as described herein.

Once operating, the CPU 902 displays a menu of programming options to the user via an LCD display 914 or another suitable computer display device. To this end, the CPU 902 may, for example, display a menu of specific programming parameters of the LP(s) 102 to be programmed or may display a menu of types of diagnostic data to be retrieved and displayed. In response thereto, the user or physician enters various commands via either a touch screen 916 overlaid on LCD display 914 or through a standard keyboard 918 supplemented by additional custom keys 920, such as an emergency VVI (EVVI) key, keys to mark a value to be used as input, or keys or buttons specifying mode (e.g. mapping, tether complete). The EVVI key sets the LP(s) 102 to a safe VVI mode with high pacing outputs. The various types of displays, keys, and other input and output devices are examples of a user interface that can be used to implement certain embodiments described herein.

Typically, the user or physician initially controls the external device 109 to retrieve data stored within one or more of the LPs 102. To this end, CPU 902 transmits appropriate signals to a telemetry circuit 922, which provides components for directly interfacing with the LP(s) 102. The telemetry subsystem 922 can include its own separate CPU 924 for coordinating the operations of the telemetry subsystem 922. The main CPU 902 of the external device 109 communicates with telemetry subsystem CPU 924 via internal bus 904. The telemetry subsystem 922 may include a telemetry circuit 926 for communicating with the LP(s) 102. Telemetry subsystem 922 may utilize one or more types of communication invention to communicate with the LP(s) 102, such as, but not limited to, conductive communication, RF communication, or inductive communication. Telemetry subsystem 922 may include a receiver (e.g. telemetry circuit 926) configured to wirelessly receive data from the LP 102, which may be communicatively coupled to one or more processors such as processor 902.

Patient and device diagnostic data stored within the LP(s) 102 can be transferred to the external device 109. Further, the LP(s) 102 can be instructed to provide measurements, sensed EGM, and/or other types of data to the external device 109. The CPU 902 can provide the model 950 to which data received from an LP 102 is provided and/or to which one or more measures of one or more features an EGM are provided. The model 950 can be used to output the indication of whether the LP 102 should be chronically implanted at a potential implant site. Such an indication can be a visual indication output via a display (e.g., LCD display 914) or touch screen (e.g., touch screen 916), and/or can be an audio indication output via a speaker (e.g., speaker 944), but is not limited thereto.

While model 950 is shown as being included within and/or implemented and/or executed by processor or CPU 902, in other embodiments, model 950 may be implemented, included within or executed by a different processor, such as a system external to or distant from external device 109, such as a cloud-based system with which external device is communicatively coupled. Further, while model 950 is shown as being included within CPU 902, in some embodiments the executable code and data for model 950 may be stored within a storage device such as one of storage devices 906, 908 and/or 930, and model 950 may be executed by CPU 902 or another processor.

The external device 109 can also include a Network Interface Card ("NIC") 960 to permit transmission of data to and from other computer systems via a router 962 and Wide Area Network ("WAN") 964. Alternatively, the external device 109 might include a modem for communication via the Public Switched Telephone Network (PSTN). Depending upon the implementation, the modem may be connected directly to internal bus 904 and may be connected to the internal bus 904 via either a parallel (I/O) port 940 or a serial (I/O) port 942. Data transmitted from other computer systems may include, for example, data regarding medication prescribed, administered, or sold to the patient.

The external device 109 receives data from the LP(s) 102, including parameters representative of the current programming state of the LP(s) 102. The external device 109 can also receive EGMs, samples thereof, and/or date indicative thereof from the LP(s) 102. Under the control of the physician, the external device 109 displays the current programming parameters and permits the physician to reprogram the parameters. To this end, the physician enters appropriate commands via any of the aforementioned input devices and, under control of the CPU 902, the programming commands are converted to specific programming parameters for transmission to the LP(S) 102 to thereby reprogram the LP(s) 102. Prior to reprogramming specific parameters, the physician may control the external device 109 to display any or all of the data retrieved from the LP(s) 102, including displays of EGMs, displays of electrodes that are candidates as cathodes and/or anodes, and statistical patient information. Any or all of the information displayed by external device 109 may also be printed using a printer 936.

A speaker 944 may be included for providing audible tones to the user, such as a warning beep in the event improper input is provided by the physician. Telemetry subsystem 922 may additionally include an input/output circuit 946 which can control the transmission of analog output signals, such as EGM signals output to an EGM machine or chart recorder. Other peripheral devices may be connected to the external device 109 via parallel port 940 or a serial port 942 as well. Although one of each is shown, a plurality of Input/Output (I/O) ports might be provided.

With the external device 109 configured as shown, a physician or other authorized user can retrieve, process, and display a wide range of information received from the LP(s) 102 and reprogram the LP(s) 102, if needed. The descriptions provided herein with respect to FIG. 9 are intended merely to provide an overview of the operation of the example external device 109 and are not intended to describe in detail every feature of the hardware and software of the device and are not intended to provide an exhaustive list of the functions performed by the device.

FIG. 10 illustrates a high level flow diagram showing a method. Referring to FIG. 10, in operation 1000, a model may be created: for example, data may be collected, and using statistical methods, AI-based training, or other methods, one or more models such as those shown in Tables 1, 2 and 3, but not limited thereto, may be created.

In operation 1010, for a newly seen patient (e.g., one not used to collect data for operation 1000), a user may use a delivery system to perform a mapping procedure with an LP (such as shown in FIG. 2) using an external device such as for example shown in FIG. 9. The user may position the LP at a mapping site within a patient, on a patient's heart, which also may be a potential implant site. Operations 1010-1060 may be performed while the delivery system is being used to position the LP at a potential implant site within the patient.

A user may input to the external device a mode or state, such as mapping or tethering, based on which the external device may perform calculations or produce one or more outputs. Before starting the mapping procedure, the user may input to the external device, e.g. using a touchscreen, keyboard, or standard keys (e.g., a "mapping" key) that a mapping mode or procedure is taking place. The external device may receive, e.g., from the LP, data such as one or more impedance measurements and/or one or more other measurements, e.g., obtained by the pacemaker or LP, other than pacing impedance. Data other than pacing impedance may include, for example, measures of one or more COI features, one or more R-wave features, and/or one or more S-wave features, but are not limited thereto. The measures of COI features can include, e.g., COI amplitude, COI AUC, maximums thereof, averages thereof, and/or the like. The measures of R-wave features can include, e.g., R-wave amplitude, R-wave upslope, R-wave peak sharpness, R-wave downslope, R-wave AUC, maximums thereof, averages thereof, and/or the like. Measures of S-wave features can include, e.g., S-wave amplitude, S-wave upslope, S-wave peak sharpness, S-wave downslope, S-wave AUC, maximums thereof, and/or averages thereof and/or the like. The external device may interpret data received from the pacemaker based on the state input or indicated by the user. It is also possible that one or more of the above mentioned measures of features of an EGM sensed by the LP can be determined by the external device based on EGM data transmitted by the LP to the external device. For example, measures of one or more COI features, one or more R-wave features, and/or one or more S-wave features, can be determined by the external device based on EGM date transmitted by the LP to the external device.

During the mapping procedure, measurements such as impedance, COI, slopes of EGM, etc., may be received and/or calculated by the external device, collected, and may be displayed to a user. Some values may alternately be determined by a user reading an external device display of underlying data. Multiple parallel values may be collected over time, e.g. COI may be collected repeatedly over time during mapping, and such values may be output over time on a rolling basis. A user may press a key, button, touchscreen indication, etc., on the external device to select a value to be used, e.g. indicating use currently displayed COI, use currently displayed impedance, etc. Alternately, a user may input, e.g. using a keyboard or touchscreen, values such as COI or impedance, to be used by the external device. A user may perform oversight, and alter values output by external device to correct, e.g. COI, or other values.

In operation 1020, the external device may output a recommendation or rating as to, or data related to, whether or not, based on mapping measurements, a tether procedure should take place. This may be performed by providing the received or calculated data from the pacemaker to a model that is produced prior to the implant procedure based on empirical data collected from other patients. In one embodiment, this is based on the example model or formulas shown in Table 3 below:

Table 3

$$Y' = CoeffA - CoeffB \times Map\_Imp - CoeffC \times Map\_COI$$
$$P(1) = \exp(Y')/1+\exp(Y'))$$

Coefficients CoeffA, CoeffB and CoeffC may be generated in accordance with various embodiments. For example, during the mapping mode as the user moves the LP across different sites, and as different heartbeats occur, the external device may collect different impedance (Map_Imp) and COI (Map_COI) measurements, for example for each such measurement once per heartbeat, once per time period (e.g., second), etc. The external device may periodically (e.g., after each heartbeat, after each time period, etc.) output (e.g. to a display) P(1), or an interpretation or rating based on P(1). For example, if P(1) is under a threshold, a "tether" rating may be output, and if not, a "no tether" rating may be output. In one embodiment mapping values are input to a model to determine where to tether; then mapping and/or tether or other values are added to the existing mapping values to produce a model recommendation regarding implanting,

If the output from the external device does not indicate tethering should take place (e.g., no tethering rating is output; or values output indicate to a user tethering should not take place), the user may move the LP to a different site and continue mapping - one or more mapping portions, and one or more testing portions may take place. While an example formula or model in Table 3 is used, other formulas may be used to determine tethering and/or implant site, such as the formula or model or a variant shown in Table 2. Output in operation 1010 and/or operation 1040 may be a probability (e.g., probabilistic indication), e.g., that positioning at the site will produce adequate capture threshold after a period of time; or predicted PCT for the position at a time in the future (e.g., three months in the future, but not limited thereto), such as by using the example model of Table 2; a predicted change or percent change in PCT after a period of time; a binary indication (e.g. implant/do not implant); or a score. While specific data is shown in examples as being collected during mapping and tether/testing portions, data such as pacing impedance or other data may be collected during mapping and/or testing, e.g. one or the other or both.

In operation 1030, the user may decide to enter a tether procedure (aka tether or testing mode), and may provide input that the tether mode is being entered to the external device, e.g., using a keyboard, touchscreen, or "tether" key or button. In one embodiment, in response to this indication, or based on other information, the last determined relevant mapping value (e.g., COI) or set of mapping values may be stored or noted by the external device for future use. The user may tether the LP (typically at the same site for which the last mapping values were captured). Alternately or in addition a tethering indication may be provided when tethering is complete: the user may then provide input to the external device that tethering is complete, e.g. using a touchscreen, keyboard, or standard key (e.g. a "tether complete" key).

In operation 1040, upon input or indication that tethering mode is entered or is complete, the external device may collect or calculate data after the LP is tethered. For example, the external device (e.g. via a receiver) may collect or receive from the LP or pacemaker data such as tether impedance, pacing impedance measurements and/or one or more measurements, e.g. obtained by the pacemaker or LP, other than pacing impedance. Typically, the values collected from tethering and mapping are based on the pacemaker being at same position on the heart, but also are typically at different times, e.g. a mapping state vs. a tethering state. While in some embodiments, some data is collected during mapping and some after tethering, in other embodiments all data may be collected during either mapping or after tethering. While in some embodiments each measurement or variable in a model is based on one measurement at one point in time, in other embodiments, some measurements may be an average over time. All or a subset, or at least a portion, of the data received from the LP or receiver may be provided to one or more models, as described elsewhere including in the operations below.

In operation 1050, the external device may output a recommendation, indication or rating as to whether or not, based on measurements such as tether and/or mapping measurements, and/or a model (such as those in Tables 1 or 2), the LP should be chronically implanted at the potential implant site. In various embodiments, this output may be determined by providing the received or calculated data to a model that is produced prior to the implant procedure based on data collected from other patients. This output may be P(1) according to the formulas in Table 1, or PCT for the LP a period of time (e.g., 3-months) following the implant (e.g., as in Table 2), or an interpretation or rating based on P(1) or another value. For example, if P(1) is below a threshold, an "implant" rating may be output; if not, a "do not implant" rating may be output.

In other embodiments, tethering measurements need not be used, and a formula, such as one described with respect to operation 1020, may be used to take mapping measurements (e.g., impedance (Map_Imp) and COI (Map_COI) measurements) to recommend that the LP be tethered and also implanted at a certain site.

In operation 1060, a user may implant the LP based on the output or recommendations.

Embodiments of the present invention have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope of the claimed invention. For example, it would be possible to combine or separate some of the steps shown in FIGS. 4 and 10. It may also be possible to reorder some of the steps shown in FIGS. 4 and 10. For another example, it is possible to change the boundaries of some of the blocks shown in FIGS. 2 and 9.

In one embodiment, a COI feature is selected from the group consisting of COI amplitude, and COI AUC. In one embodiment, a measure of an R-wave feature is selected from the group consisting of R-wave amplitude, R-wave upslope, R-wave peak sharpness, R-wave downslope, and R-wave AUC. In one embodiment, a measure of S-wave features is selected from the group consisting of S-wave amplitude, S-wave upslope, S-wave peak sharpness, S-wave downslope, and S-wave AUC. In one embodiment, a measure of feature of the EGM sensed by the LP is determined for the EGM sensed by the LP during a portion selected from the group consisting of the mapping portion of an implant procedure and the testing portion of an implant procedure.

An embodiment relates to a system 100 for use during an implant procedure used to implant a LP 102a, 102b within a patient. The LP 102a, 102b including a fixation mechanism 205 and a distal electrode 108a located at a distal end of the LP 102a, 102b and also including a further electrode 108b. The implant procedure including one or more mapping portions and one or more testing portions. During each said mapping portion of the implant procedure the distal electrode 108a is in contact with the potential implant site and the fixation mechanism 205 is not affixed to the potential implant site. The system 100 comprises a receiver 922 configured to wirelessly receive, from the LP 102a, 102b, data comprising one or more pacing impedance measurements and at least one measurement obtained by the LP other than pacing impedance. The system 100 also comprises one or more processors 924, 901 communicatively coupled to the receiver 922. The one or more processors 924, 902 configured to provide at least a portion of the data received by the receiver 922 to a model 950 that is produced prior to the implant procedure based on data collected from other LPs implanted in other patients, and use the model 950 to output an indication of whether the LP 102a, 102b should be chronically implanted at a potential implant site.

In an embodiment, the data comprises at least one of the following one or more COI features, one or more R-wave features, or one or more S-wave features.

In an embodiment, the data comprises at least one of the following slope of an upslope of an R-wave, or slope of a downslope of the R-wave.

In an embodiment, during each said testing portion of the implant procedure the distal electrode 108a is in contact with the potential implant site and the fixation mechanism 205 is affixed to the potential implant site. In this embodiment, at least some of the data that is received from the LP 102a, 102b, provided to the model 950, and used by the model 950 to output the indication, is obtained by the LP 102a, 102b during at least one of the one or more mapping portions and/or comprises one or more measures of one or more features sensed by the LP 102a, 102b during the at least one of the one or more mapping portions. Further, in this embodiment, at least some of the data that is received from the LP 102a, 102b, provided to the model 950, and used by the model 950 to output the indication, is obtained by the LP 102a, 102b during at least one of the one or more testing portions and/or comprises one or more features sensed by the LP 102a, 102b during the at least one of the one or more testing portions.

In an embodiment, the one or more pacing impedance measurements is/are measured during at least one of the one or more mapping portions of the implant procedure, or during at least one of the one or more testing portions of the implant procedure, or both.

In an embodiment, the model 950 comprises a linear regression model.

In a particular embodiment, the linear regression model comprises a univariate or multivariate linear regression model.

In another embodiment, the model 950 comprises a logistic regression model.

In a further embodiment, the model 950 comprises a ML model that is trained based on the data collected from other LPs implanted in other patients prior to the implant procedure.

In yet another embodiment, the model 950 comprises a binary classifier model configured to output a binary indication.

In an embodiment, the indication output by the model 950, of whether the LP 102a, 102b should be chronically implanted at the potential implant site, comprises a probabilistic indication. Alternatively, or in addition, the indication output by the model 950, of whether the LP 102a, 102b should be chronically implanted at the potential implant site, comprises a predicted pacing capture threshold for the LP (102a, 102b) a period of time in the future if the LP 102a, 102b were chronically implanted at the potential implant site.

In an embodiment, the system 100 comprises an external programmer 109 that includes the receiver 922 and the one or more processors 924, 901 communicatively coupled to the receiver 922.

In an embodiment, the model 950 is implemented by at least one of the one or more processors 924, 902 of the system 100.

In an embodiment, the model 950 is implemented by a processor other than the one or more processors 924, 902 of the system 100.

An embodiment relates to a non-transitory computer readable medium comprising a plurality of instructions stored thereon and executable by at least one processor 924, 902 of an external system 100 during an implant procedure used to implant a LP 102a, 102b within a patient. The plurality of instructions comprising instructions for providing, to a model 950, data received during the implant procedure from the LP 102a, 102b comprising one or more pacing impedance measurements and at least one measurement obtained by the LP 102a, 102b other than pacing impedance. The model 950 that is produced prior to the implant procedure based on data collected from other LPs implanted in other patients. The plurality of instructions also comprising instructions for using the model 950 to output an indication of whether the LP 102a, 102b should be chronically implanted at a potential implant site.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments may include different combinations of features noted in the described embodiments, and features or elements described with respect to one embodiment or flowchart can be combined with or used with features or elements described with respect to other embodiments.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present invention without departing from its scope. While the dimensions, types of materials and coatings described herein are intended to define the parameters of the embodiments of the present invention, they are by no means limiting and are exemplary embodiments. Many other embodiments will be apparent to those of skill in the art upon reviewing the above description. The scope of the embodiments of the present invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

**Claims**

1. A system (100) for use during an implant procedure used to implant a leadless pacemaker, LP, (102a, 102b) within a patient, the LP (102a, 102b) including a fixation mechanism (205) and a distal electrode (108a) located at a distal end of the LP (102a, 102b) and also including a further electrode (108b); the implant procedure including one or more mapping portions and one or more testing portions; wherein during each said mapping portion of the implant procedure the distal electrode (108a) is in contact with the potential implant site and the fixation mechanism (205) is not affixed to the potential implant site; the system (100) comprising:

   a receiver (922) configured to wirelessly receive, from the LP (102a, 102b), data comprising one or more pacing impedance measurements and at least one measurement obtained by the LP other than pacing impedance; and one or more processors (924, 901) communicatively coupled to the receiver (922); the one or more processors (924, 902) configured to:

   provide at least a portion of the data received by the receiver (922) to a model (950) that is produced prior to the implant procedure based on data collected from other LPs implanted in other patients; and use the model (950) to output an indication of whether the LP (102a, 102b) should be chronically implanted at a potential implant site.

2. The system (100) of claim 1, wherein the data comprises at least one of the following:

   one or more current of injury (COI) features;
   one or more R-wave features; or
   one or more S-wave features.

3. The system (100) of any one of claims 1 or 2, wherein the data comprises at least one of the following:

   slope of an upslope of an R-wave; or
   slope of a downslope of the R-wave.

4. The system (100) of any one of claims 1 through 3, wherein:

   during each said testing portion of the implant procedure the distal electrode (108a) is in contact with the potential implant site and the fixation mechanism (205) is affixed to the potential implant site;
   at least some of the data that is received from the LP (102a, 102b), provided to the model (950), and used by the model (950) to output the indication, is obtained by the LP (102a, 102b) during at least one of the one or more mapping portions and/or comprises one or more measures of one or more features sensed by the LP (102a, 102b) during the at least one of the one or more mapping portions; and
   at least some of the data that is received from the LP (102a, 102b), provided to the model (950), and used by the model (950) to output the indication, is obtained by the LP (102a, 102b) during at least one of the one or more testing portions and/or comprises one or more features sensed by the LP (102a, 102b) during the at least one of the one or more testing portions.

5. The system (100) of any one of claims 1 through 4, wherein the one or more pacing impedance measurements is/are measured during at least one of the one or more mapping portions of the implant procedure, or during at least one of the one or more testing portions of the implant procedure, or both.

6. The system (100) of any one of claims 1 through 5, wherein:
   the model (950) comprises a linear regression model.

7. The system (100) of claim 6, wherein:
   the linear regression model comprises a univariate or multivariate linear regression model.

8. The system (100) of any one of claims 1 through 5, wherein:
   the model (950) comprises a logistic regression model.

9. The system (100) of any one of claims 1 through 5, wherein:
   the model (950) comprises a machine learning (ML) model that is trained based on the data collected from other LPs implanted in other patients prior to the implant procedure.

10. The system (100) of any one of claims 1 through 5, wherein:
    the model (950) comprises a binary classifier model configured to output a binary indication.

11. The system (100) of any one of claims 1 through 9, wherein:

    the indication output by the model (950), of whether the LP (102a, 102b) should be chronically implanted at the potential implant site, comprises a probabilistic indication; or
    the indication output by the model (950), of whether the LP (102a, 102b) should be chronically implanted at the potential implant site, comprises a predicted pacing capture threshold for the LP (102a, 102b) a period of time in the future if the LP (102a, 102b) were chronically implanted at the potential implant site.

12. The system (100) of any one of claims 1 through 11, wherein the system (100) comprises an external programmer (109) that includes the receiver (922) and the one or more processors (924, 901) communicatively coupled to the receiver (922).

13. The system (100) of any one of claims 1 through 12, wherein the model (950) is implemented by at least one of the one or more processors (924, 902) of the system (100).

14. The system (100) of any one of claims 1 through 12, wherein the model (950) is implemented by a processor other than the one or more processors (924, 902) of the system (100).

15. A non-transitory computer readable medium comprising a plurality of instructions stored thereon and executable by at least one processor (924, 902) of an external system (100) during an implant procedure used to implant a leadless pacemaker, LP, (102a, 102b) within a patient, the plurality of instructions comprising:

instructions for providing, to a model (950), data received during the implant procedure from the LP (102a, 102b) comprising one or more pacing impedance measurements and at least one measurement obtained by the LP (102a, 102b) other than pacing impedance, wherein the model (950) that is produced prior to the implant procedure based on data collected from other LPs implanted in other patients; and
instructions for using the model (950) to output an indication of whether the LP (102a, 102b) should be chronically implanted at a potential implant site.

**FIG. 1**

**FIG. 2**

**FIG. 3**

Provide model produced based on empirical data collected from other LPs implanted in other patients — 402

Receive, from LP,
data including measurement(s) obtained by LP and
EGM(s) sensed by LP
while LP is positioned at potential implant site — 404

Determine measure(s) of feature(s) of EGM(s) sensed by LP — 406

Provide to model
data received from LP and measure(s) of EGM(s) — 408

Use model to output indication,
based on data received from LP and/or measure(s) of EGM(s),
of whether LP should remain chronically implanted
at potential implant site — 410

Indication output that LP should remain chronically implanted at potential implant site? — 412

No

Yes

End

LP repositioned to be at new potential implant site

## FIG. 4

| Feature | p-value |
|---|---|
| **Mapping** | |
| **Impedance** | **.04** |
| R-wave amplitude | .46 |
| R-wave upslope | .95 |
| R-wave peak sharpness | **.05** |
| S-wave amplitude | .39 |
| Downstroke (S-wave) | .51 |
| COI amplitude | .09 |
| **Tether** | |
| **Impedance** | **<0.01** |
| R-wave amplitude | **.03** |
| R-wave upslope | .49 |
| R-wave peak sharpness | .10 |
| S-wave amplitude | .74 |
| Downstroke (S-wave) | .14 |
| COI amplitude | .08 |

# FIG. 5

## FIG. 6

ROC for Mapping COI + Mapping Impedance

## FIG. 7

ROC for Mapping COI + Tether Impedance

## FIG. 8

**FIG. 9**

```
┌─────────────────────────────────────────────────────────────┐
│                       Create Model                          │──1000
└─────────────────────────────────────────────────────────────┘
                              │
                              ▼
┌─────────────────────────────────────────────────────────────┐
│                     Perform Mapping                         │──1010
└─────────────────────────────────────────────────────────────┘
                              │
                              ▼
┌─────────────────────────────────────────────────────────────┐
│        Output Recommendation/Rating related to Tether       │──1020
└─────────────────────────────────────────────────────────────┘
                              │
                              ▼
┌─────────────────────────────────────────────────────────────┐
│                      Perform Tether                         │──1030
└─────────────────────────────────────────────────────────────┘
                              │
                              ▼
┌─────────────────────────────────────────────────────────────┐
│                  Collect Post-Tether Data                   │──1040
└─────────────────────────────────────────────────────────────┘
                              │
                              ▼
┌─────────────────────────────────────────────────────────────┐
│     Output Recommendation/Rating related to Implantation    │──1050
└─────────────────────────────────────────────────────────────┘
                              │
                              ▼
┌─────────────────────────────────────────────────────────────┐
│                   Perform Implantation                      │──1060
└─────────────────────────────────────────────────────────────┘
```

# *FIG. 10*

Europäisches
Patentamt

European
Patent Office

Office européen
des brevets

## EUROPEAN SEARCH REPORT

**Application Number**

EP 25 21 5479

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/077022 A1 (GRINBERG YANINA [US] ET AL) 18 March 2021 (2021-03-18) * paragraphs [0006], [0030], [0055], [0064], [0089], [0121], [0133], [0134]; figures 1,2b * ----- | 1-15 | INV. A61B5/06 A61B5/00 A61N1/365 A61N1/372 |
| A | US 2008/243202 A1 (PATANGAY ABHILASH [US] ET AL) 2 October 2008 (2008-10-02) * paragraphs [0016], [0033], [0040], [0044], [0060], [0063]; figure 10 * ----- | 1-15 | |
| A | US 10 143 847 B1 (EDMONSON JONATHAN D [US] ET AL) 4 December 2018 (2018-12-04) * column 8, line 44 - line 67; figures 1b,10 * ----- | 1-15 | |

**TECHNICAL FIELDS SEARCHED (IPC)**

A61B
A61N

The present search report has been drawn up for all claims

| Place of search | Date of completion of the search | Examiner |
|---|---|---|
| Munich | 13 March 2026 | Franz, Volker |

EPO FORM 1503 03.82 (P04C01)

# ANNEX TO THE EUROPEAN SEARCH REPORT
## ON EUROPEAN PATENT APPLICATION NO.

EP 25 21 5479

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2026

| Patent document cited in search report | | Publication date | Patent family member(s) | | Publication date |
|---|---|---|---|---|---|
| US 2021077022 A1 | | 18-03-2021 | CN | 114390935 A | 22-04-2022 |
| | | | US | 2021077022 A1 | 18-03-2021 |
| | | | WO | 2021050854 A1 | 18-03-2021 |
| US 2008243202 A1 | | 02-10-2008 | EP | 2142251 A1 | 13-01-2010 |
| | | | JP | 5166446 B2 | 21-03-2013 |
| | | | JP | 2010516431 A | 20-05-2010 |
| | | | US | 2008243202 A1 | 02-10-2008 |
| | | | US | 2012232607 A1 | 13-09-2012 |
| | | | WO | 2008121185 A1 | 09-10-2008 |
| US 10143847 B1 | | 04-12-2018 | NONE | | |

EPO FORM P0459

For more details about this annex : see Official Journal of the European Patent Office, No. 12/82